Europäisches Patentamt

European Patent Office

Office européen des brevets

(19)

(11) Veröffentlichungsnummer: **0 294 314**
**A2**

# (12) EUROPÄISCHE PATENTANMELDUNG

(21) Anmeldenummer: **88730119.0**

(22) Anmeldetag: **18.05.88**

(51) Int. Cl.⁴: **A 61 B 5/10**

(30) Priorität: **05.06.87 DE 3719381**

(43) Veröffentlichungstag der Anmeldung:
**07.12.88 Patentblatt 88/49**

(84) Benannte Vertragsstaaten:
**AT BE CH DE ES FR GB GR IT LI LU NL SE**

(71) Anmelder: **SCHERING AKTIENGESELLSCHAFT Berlin
und Bergkamen
Müllerstrasse 170/178 Postfach 65 03 11
D-1000 Berlin 65 (DE)**

(72) Erfinder: **Chwalisz, Krzysztof, Dr.
Luzerner Strasse 1 d
D-1000 Berlin 45 (DE)**

**Kolberg, Reiner
Ludolfingerweg 43 c
D-1000 Berlin 28 (DE)**

**Niemeyer, Dieter
Hubertusallee 36
D-1000 Berlin 33 (DE)**

**Voigt, Claude-Dietrich, Dipl.-Ing.
Seestrasse 44 a
D-1000 Berlin 65 (DE)**

(74) Vertreter: **Maikowski, Michael, Dipl.-Ing. Dr.
Xantener Strasse 10
D-1000 Berlin 15 (DE)**

(54) **Cervimeter.**

(57) Die Erfindung betrifft ein Cervimeter zur Messung der radialen Dehnung der uterinen Cervix. An radialen Gewindespindeln 3, die über ein Getriebe 2 von einer Motor-Tacho-Einheit 1 angetrieben werden, sind Führungsschlitten 4 geführt, die Spreizstifte 5 tragen. Diese Konstruktion ermöglicht eine exakte parallele radiale Bewegung der Spreizfinger 11, die an den Spreizstiften 5 befestigt sind.

Fig.1

EP 0 294 314 A2

**Beschreibung**

**Cervimeter**

Die Erfindung geht aus von einem Cervimeter nach dem Oberbegriff des Patentanspruchs 1.

Sowohl für den behandelnden als auch für den forschenden Arzt ist die Untersuchung der physiologischen Cervixreifung von großer Bedeutung. Zu diesem Zweck ist es erforderlich, die Dehnbarkeit und Dilatation des cervikalen Gewebes in vivo zu quantifizieren. Aus der US-PS 44 32 376 ist ein Zangengerät bekannt, das von der Hand bedient wird. Dieses Zangengerät weist zwei Zangenbacken auf, mit denen der Cervixlippenrand erfaßt und auf diesen ein Kompressionsdruck ausgeübt wird. Im Griffbereich weist dieses Gerät zwei Geber auf; einen Distanzgeber und einen Kraftgeber. Der Distanzgeber spricht auf den Abstand der Zangenschenkel oder auf die Verdrehung der Schenkel im Gelenk an. Der Kraftgeber spricht auf die Verbiegung eines Biegungsabschnittes des Gerätes an. Mit diesem bekannten Gerät kann die Dehnung des Cervixgewebes nicht direkt sondern nur indirekt gemessen werden, da keine Dehnungsgrößen sondern Kompressionsgrößen gemessen werden. Aus der technischen Mechanik ist es bekannt, daß Kompression und Dehnung in einem bestimmten Verhältnis zueinander stehen. Dieses Verhältnis ist jedoch bei biologischen Geweben keine einfache Funktion, so daß mit diesem Gerät nur Näherungswerte ermittelt werden können. Dieses bekannte Gerät ist kein Präzisionsinstrument, da mit diesem zeitliche Abläufe nicht in vorbestimmter Weise durchgeführt werden können.

Der vorliegenden Erfindung liegt die Aufgabe zu Grunde, ein Cervimeter zu schaffen, mit dem die gleichzeitige Dehnbarkeit in drei radialen Richtungen der uterinen Cervix mit beliebigen vorbestimmten Kraft-, Weg- und Zeitabhängigkeiten durchgeführt werden kann.

Erfindungsgemäß wird diese Aufgabe durch die technische Lehre des Patentanspruchs 1 gelöst.

In vorteilhafter Weise werden über eine Motor-Tacho-Einheit die Spreizstifte exakt parallel zueinander linear radial bewegt, so daß eine genaue radiale Dehnung möglich ist. Über die Motor-Tacho-Einheit kann in Verbindung mit der Steigung der Weg gemessen werden und die Widerstandskräfte, die einer der Spreizstifte überwinden muß, werden auf einen Kraftaufnehmer übertragen. Es besteht die Möglichkeit, eine Dehnungsgeschwindigkeit vorzugeben. Ferner kann eine maximale Dehnungskraft eingestellt werden und weiter ist es möglich, eine geometrische Dehnungsgröße vorzubestimmen. Daneben sind alle Kombinationen dieser Meßtechniken möglich. In einfachster Weise kann bei diesem Cervimeter eine elektronische Steuerung vorgesehen sein, mit der sich die Meßwerte aus Weg, Kraft und Zeit bestimmen lassen.

Ein besonders einfacher Aufbau ergibt sich, wenn das Getriebe ein Kegelradgetriebe ist. Mit Vorteil haben die Gewindespindeln einen Winkelabstand von 120° voneinander.

Damit auf die Gewindespindeln keinen Reaktionskräfte übertragen werden, sind die Führungsschlitten gleitbar in einem Gehäuse geführt. Mit Vorteil können dabei die Führungsschlitten auf beiden Seitenflächen Schwalbenschwänze aufweisen, die in Schwalbenschwanzführungen im Gehäuse eingreifen.

Die Spreizstifte, die auswechselbar sind, tragen an ihren Enden Spreizfinger, die in die uterine Cervix eingeführt werden, um diese zu dehnen.

Der schwenkbar gelagerte Spreizstift kann an seinem Führungsschlitten mittels eines Hebelabschnittes gelagert sein, der zwischen seiner Drehachse und dem Spreizstift eine Kraft auf einen Hebelarm ausübt.

Mit besonderem Vorteil kann der Kraftaufnehmer ein Kraftaufnahmering sein, der im Gehäuse über dem Getriebe angeordnet ist und einen Zapfen aufnimmt, der am Ende des die Kraft aufnehmenden Hebelarms befestigt ist. Dieser Hebelarm kann insbesondere mit einem Ende im Gehäuse frei drehbar gelagert sein und mit Spiel den Führungsschlitten mit dem verschwenkbaren Spreizstift durchsetzen. Dabei kann der Hebelabschnitt eine gegen den Hebelarm anliegende Druckrolle zur Übertragung der Kraft aufweisen.

Ein Ausführungsbeispiel der Erfindung soll in der folgenden Beschreibung unter Bezugnahme auf die Fig. der Zeichnung erläutert werden.

Es zeigen:

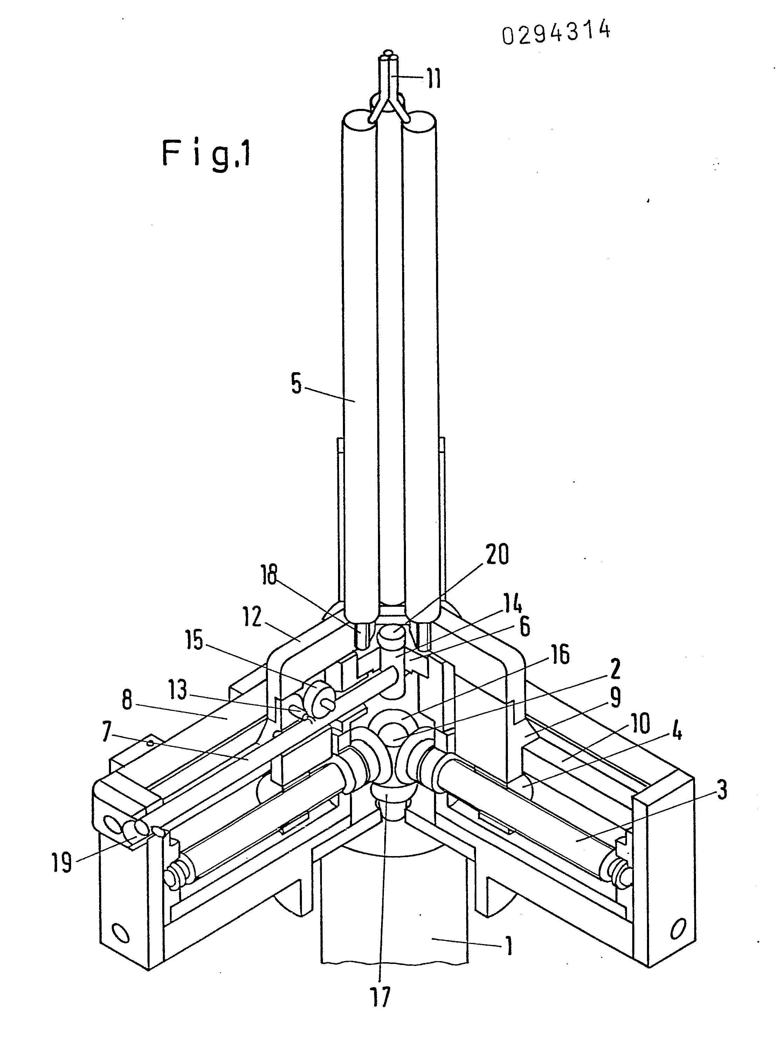

Fig. 1 eine teilweise geschnittene Ansicht des Cervimeters

und

Fig. 2 eine vergrößerte Detailansicht des in Fig. 1 gezeigten Cervimeters.

In einem dreiarmigen Gehäuse 8 sind radiale Gewindespindeln 3 drehbar montiert. Diese radialen Gewindespindeln 3 haben einen Winkelabstand von 120 voneinander. An ihren inneren Enden tragen diese radialen Gewindespindeln 3 Kegelräder 16, die mit einem Kegelrad 17 unter Bildung eines Getriebes 2 kämmen, welches auf der Abtriebswelle einer Motor-Tacho-Einheit 1 sitzt. Im Gehäuse 8 sind Schwalbenschwanzführungen 10 ausgebildet. In diesen Schwalbenschwanzführungen 10 sind mittels Schwalbenschwänzen 9 Führungsschlitten 4 linear geführt. Die Führungsschlitten 4 haben auf beiden Seitenflächen Schwalbenschwänze 9, die in entsprechende Schwalbenschwanzführungen 10 eingreifen. Jeder dieser Führungsschlitten 4 steht in Antriebsverbindung mit einer radialen Gewindespindel 3 und trägt einen Spreizstift 5. Die Spreizstifte 5 tragen an ihren Enden Spreizfinger 11, die in die uterine Cervix eingeführt werden können.

Bei dieser Konstruktion werden über die Motor-Tacho-Einheit 1 und das Getriebe 2 die radialen Gewindespindeln 3 angetrieben. Dadurch werden die Führungsschlitten radial auseinander- oder zusammengefahren und nehmen dabei die Spreizstifte 5 mit ihren Spreizfingern 11 mit. Die Spreizstifte 5 sind mittels Zapfen 18 auswechselbar in den zugeordneten Führungsschlitten 4 gelagert.

Aus Motordrehzahl und der Steigung der radialen Gewindespindeln 3 kann die Wegstrecke bestimmt werden, um die die Führungsschlitten 4 bewegt werden. Bei dieser Bewegung werden die Spreizfinger 11 exakt parallel zueinander auseinandergefahren, so daß eine exakte radiale Dehnung der uterinen Cervix möglich ist.

Um die auftretenden Kräfte bestimmen zu können, ist einer der Spreizstifte 5 an seinem Führungsschlitten 4 mittels eines Hebelabschnittes 12 gelagert. Bei 13 ist die Dreh oder Schwenkachse dieses Hebelabschnittes 12 dargestellt. Zwischen dieser Drehachse 13 und dem Spreizstift 5 ist am Hebelabschnitt 12 eine Druckrolle 15 drehbar gelagert. Diese Druckrolle 15 liegt gegen einen Hebelarm 7 an, der im Gehäuse 8 bei 19 frei drehbar gelagert ist.

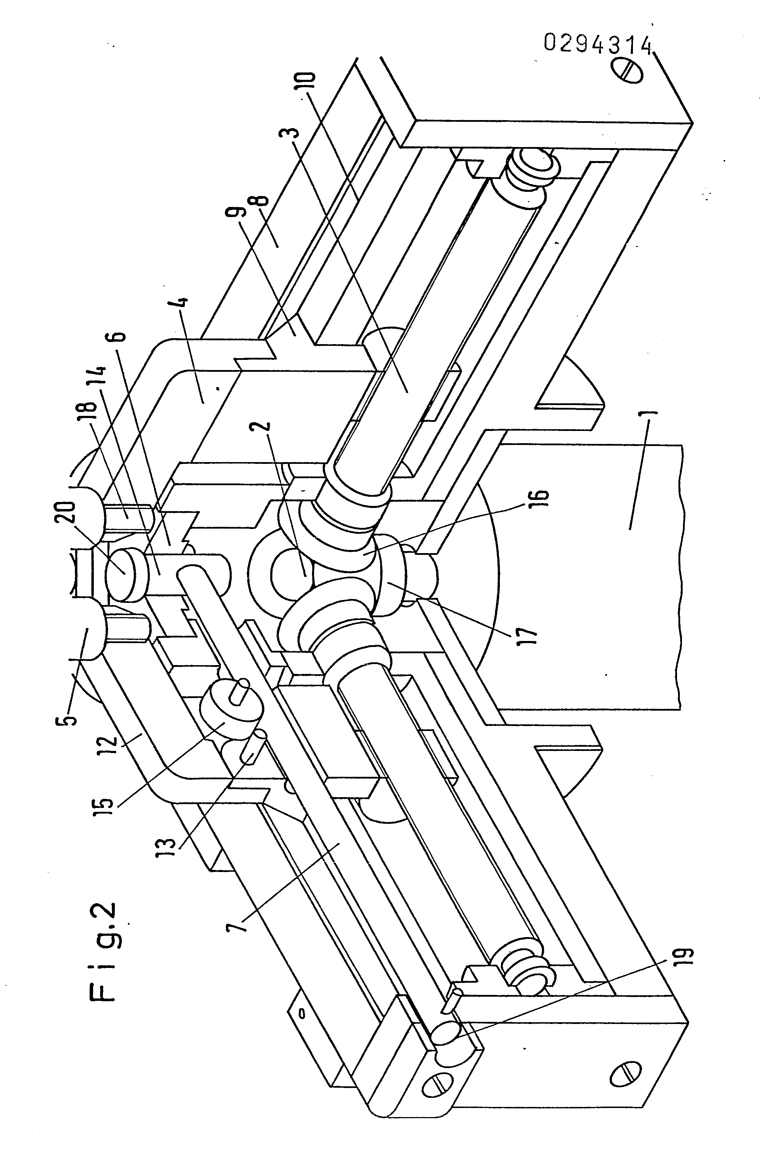

Wie Fig. 2 zeigt, durchsetzt dieser Hebelarm 7 den Führungsschlitten 4 mit Spiel und trägt an seinem Ende einen Zapfen 14. Dieser Zapfen 14 erstreckt sich durch die Mittelbohrung eines Kraftaufnehmers 6, er im Gehäuse 8 oberhalb des Getriebes 2 eingespannt ist. Dieser Zapfen 14 weist einen Kopf 20 auf, der gegen den Kraftaufnehmer 6 anliegt.

Wenn die Spreizstifte 5 in der beschriebenen Weise auseinandergefahren werden, überträgt der eine Spreizstift 5 ein Moment auf den Hebelabschnitt 12. Dieses Moment erzeugt eine Verdrehung des Hebelabschnittes 12 um seine Drehachse 13. Dadurch kann die Druckrolle 15 eine Kraft auf den Hebelarm 7 ausüben. Diese Kraft wird über den Zapfen 18 und den Kopf 20 auf den Kraftaufnehmer 6 übertragen.

## Patentansprüche

1. Cervimeter zur Messung der Dehnungseigenschaften des Muttermundes in vivo mit
   1. einer Längenmeßeinrichtung und
   2. einer Kraftmeßeinrichtung, gekennzeichnet, durch
   3. eine Motor-Tacho-Einheit (1), die
   4. über ein Getriebe (2)
   5. drei radial angeordnete Gewindespindeln (3) antreibt, die
   6. in Antriebsverbindung mit Führungsschlitten (4) stehen, die
   7. Spreizstifte (5) tragen, von denen
   8. einer an dem zugeordneten Führungsschlitten verschwenkbar gelagert ist, um eine Kraft auf einen Kraftaufnehmer (6) zu übertragen.

2. Cervimeter nach Anspruch 1, dadurch gekennzeichnet, daß
   4.1 das Getriebe (2) ein Kegelradgetriebe ist.

3. Cervimeter nach einem der Ansprüche 1 oder 2, dadurch gekennzeichnet, daß
   5.1 die radialen Gewindespindeln (3) einen Winkelabstand von 120 voneinander haben.

4. Cervimeter nach einem der Ansprüche 1 - 3, dadurch gekennzeichnet, daß
   6.1 die Führungsschlitten (4) gleitbar in einem Gehäuse (8) geführt sind.

5. Cervimeter nach Anspruch 4, dadurch gekennzeichnet, daß
   6.1.1 die Führungsschlitten (4) auf den beiden Seitenflächen Schwalbenschwänze (9) aufweisen, die in Schwalbenschwanzführungen (10) im Gehäuse (8) eingreifen.

6. Cervimeter nach einem der Ansprüche 1 - 5, dadurch gekennzeichnet, daß
   7.1 die Spreizstifte (5) an ihren Enden Spreizfinger (11) tragen.

7. Cervimeter nach einem der Ansprüche 1 - 6, dadurch gekennzeichnet, daß
   8.1 der schwenkbar gelagerte Spreizstift (5) an dem zugeordneten Führungsschlitten (4) mittels eines Hebelabschnittes (12) gelagert ist, der
   8.1.1 zwischen seiner Drehachse (13) und dem Spreizstift (5) eine Kraft auf einen Hebelarm (7) ausübt.

8. Cervimeter nach einem der Ansprüche 1 - 7, dadurch gekennzeichnet, daß
   7.2 die Spreizstifte (5) auswechselbar sind.

9. Cervimeter nach einem der Ansprüche 7 oder 8, dadurch gekennzeichnet,
   8.2 daß der Kraftaufnehmer (6) ein Kraftaufnahmering ist, der
   8.2.1 im Gehäuse (8) über dem Getriebe (2) angeordnet ist und
   8.2.2 einen Zapfen (14) aufnimmt, der
   8.2.3 am Ende des Hebelarms (7) befestigt ist.

10. Cervimeter nach einem der Ansprüche 4 - 9, dadurch gekennzeichnet, daß
    8.3 der Hebelarm (7) mit einem Ende im Gehäuse (8) frei drehbar (19) gelagert ist und
    8.3.1 mit Spiel den Führungsschliten (4) mit dem verschwenkbaren Spreizstift (5) durchsetzt.

11. Cervimeter nach Anspruch 7, dadurch gekennzeichnet, daß
    8.4 der Hebelabschnitt (12) eine gegen den Hebelarm (7) anliegende Druckrolle (15) trägt.

Fig.1

0294314

Fig.2